# EUROPEAN PATENT APPLICATION

(11) **EP 4 539 057 A1**
(43) Date of publication of application: **16.04.2025**
(21) Application number: 23203446.2
(22) Date of filing: 13.10.2023
(51) Int. Cl.: G16H 30/40

(54) **ANNOTATING MEDICAL IMAGES**

(71) Applicant: Koninklijke Philips N.V., 5656 AG Eindhoven (NL)
(72) Inventor: KOEPNICK, Johannes, Eindhoven (NL); LUNDT, Bernd, Eindhoven (NL); GOOßEN, Andre, 5656AG Eindhoven (NL); MAY, Jan Marek, Eindhoven (NL); RONGE, Svenja, Eindhoven (NL); STROMMER, Veronika, Eindhoven (NL); HINGENITZ, Nina, Eindhoven (NL); KUEHL, Nicole, Eindhoven (NL)
(74) Representative: Philips Intellectual Property & Standards

(57) **Abstract**

Manual marker placement in medical imaging is disruptive to the workflow and prone to error. There is therefore provided a computer-implemented method for annotating medical images. The method comprises: obtaining a medical image (300) to be annotated; applying an annotation algorithm to the medical image, wherein the annotation algorithm determines values of one or more parameters for a post-acquisition annotation (302) to be applied to the medical image and annotates the medical image by applying the post-acquisition annotation to the medical image using the determined values, wherein the one or more parameters comprise image coordinates for a position of the post-acquisition annotation in the medical image; and outputting the annotated medical image. The method improves the workflow by automating the marker placement.

## Description

### FIELD OF THE INVENTION

The invention relates to methods and systems for annotating medical images.

### BACKGROUND OF THE INVENTION

In medical imaging, the patient's anatomy can be placed in different orientations and can be of different laterality. For example, in anterior to posterior (AP) radiographs, posterior structures are closer to the detector, and in posterior to anterior (PA) images, anterior structures are closer to the detector. Laterality refers to which side of the body that is considered, i.e., left or right. A person viewing an X-ray image without prior knowledge may not be able to identify the orientation or laterality of the patient's anatomy. Therefore, the image should be annotated as part of the examination. Typically, the radiographer annotates the image manually using a pre-acquisition marker (e.g., a lead marker indicating L=left or R=right placed in the field of view) or a post-acquisition marker that is placed in the image using software. In either case, the marker placement is disruptive to the radiography workflow and prone to error. Moreover, the marker placement may be subject to hospital-specific guidelines governing the location and/or size of the marker.

### SUMMARY OF THE INVENTION

To better address one or more of these concerns, there is provided, in a first aspect of the invention, a computer-implemented method for annotating medical images. The method comprises:
obtaining a medical image to be annotated;
applying an annotation algorithm to the medical image, wherein the annotation algorithm determines values of one or more parameters for a post-acquisition annotation to be applied to the medical image and annotates the medical image by applying the post-acquisition annotation to the medical image using the determined values, wherein the one or more parameters comprise image coordinates for a position of the post-acquisition annotation in the medical image; and
outputting the annotated medical image.

The one or more parameters may further comprise a scaling factor for the post-acquisition annotation. The one or more parameters may further comprise a brightness of the post-acquisition annotation. The one or more parameters may further comprise an orientation or rotation of the post-acquisition annotation, such as a horizontal orientation or a vertical orientation. The one or more parameters may further comprise a text direction of the annotation, such as a left-to-right script or right-to-left script.

The annotation algorithm may determine the values of the one or more parameters based on predetermined criteria. For example, the annotation algorithm may determine the values of the one or more parameters based at least partially on a type of the post-acquisition annotation. The type of the annotation may correspond to one or more of the following: a laterality marker; a view position (orientation) marker; an operator ID marker. For example, the annotation algorithm may determine the size of the post-acquisition annotation according to its type and/or diagnostic relevance, with for example a laterality or orientation marker being larger than an operator ID marker. The annotation algorithm may determine the values of the one or more parameters based at least partially on image content. For example, the annotation algorithm may determine the values of the one or more parameters to avoid overlap of the post-acquisition annotation with at least one pre-identified region of the medical image which depicts diagnostically relevant structures and/or foreign objects. In another example, the annotation algorithm may determine the values of the one or more parameters to place the post-acquisition annotation in at least one direct radiation area of the medical image. Different priorities may be assigned to different criteria, with for example the avoidance of overlap with diagnostically relevant structures assuming the highest priority. In the case of brightness as a parameter, the annotation algorithm may determine the brightness of the post-acquisition annotation based at least partially on a background brightness of the medical image. In this case, the predetermined criteria may enable the brightness to be set such that the annotation is not overly bright but still visually distinguishable from the background. In an example, using orientation as a parameter, the annotation algorithm may be configured to rotate an ordinarily-horizontal annotation into a vertical orientation to avoid overlap with the at least one pre-identified region, for example in the case of a chest examination in which a relatively small area of direct radiation leaves little room for horizontal annotations.

Applying the post-acquisition annotation to the medical image using the determined values may comprise any appropriate technique for direct and/or indirect application of the annotation to the image. In one example, the values determined by the annotation algorithm may be stored together with the post-acquisition annotation in metadata for the medical image, for later use in displaying the annotation as an overlay on a viewing station, for example. Additionally or alternatively, the annotation algorithm may render the post-acquisition annotation directly into the medical image using the determined values of the one or more parameters, which may be useful in the case that the annotated image is printed. Different classes may be assigned to different types of annotation, with some annotations being burned into the image and others provided as part of the metadata so as to be available on demand.

The annotation algorithm may utilize one or more conventional and/or AI-based techniques for determining the values. The annotation algorithm may utilize a trained machine learning model to determine the values of the one or more parameters. Additionally or alternatively, the annotation algorithm may utilize a model of anatomy depicted in the medical image to determine the image coordinates for the position of the post-acquisition annotation based at least partially on one or more landmarks comprised in the model. In this case, the annotation algorithm may further utilize prior knowledge about an orientation and/or laterality of the anatomy to determine the image coordinates. Additionally or alternatively, the annotation algorithm may utilize an image processing algorithm to select the values of the one or more parameters which avoid overlap of the post-acquisition annotation with at least one pre-identified region of the medical image which depicts diagnostically relevant structures and/or foreign objects. Any combination of such techniques may be used by the annotation algorithm to determine the values of the one or more parameters.

The annotation algorithm may utilize entropy in the determination of the one or more values. The annotation algorithm may identify at least one region of the medical image which depicts diagnostically relevant structures based on entropy within the medical image. The annotation algorithm may identify at least one direct radiation area of the medical image based on entropy within the medical image, wherein the at least one direct radiation area comprises suitable image coordinates for the position of the post-acquisition annotation.

Obtaining the medical image to be annotated may comprise acquiring the image using a medical image system or receiving a pre-acquired image. The medical image may be acquired using any appropriate medical imaging modality, but especially those in which standard annotations are required. For example, the medical image may be an X-ray image acquired using an X-ray imaging system (e.g., a mobile or fixed radiographic system or a fluoroscopic system). In other examples, the medical imaging system used to acquire the image may be a magnetic resonance imaging (MRI) system, a positron emission tomography (PET) system, an image-guided hyperthermia (IGHT) system, a single-photon emission computed tomography (SPECT) system, a computed tomography (CT) system, an ultrasound (US) imaging system, or a tomographic medical imaging system. In yet other examples, the medical image may be acquired using multiple imaging modalities. The medical image may be two- or three-dimensional.

Outputting the annotated medical image may comprise displaying the image on a user interface, storing the image for later display, or transmitting the image for display or storage.

The image coordinates may define a location inside the medical image, for example in terms of axis values (i.e., an X-axis value, a Y-axis value, and optionally also a Z-axis value) defining a position within the medical image with respect to a predetermined origin, for example one corner of the medical image. The axis values may be expressed in terms of pixels or any other appropriate unit for defining spatial locations.

The post-acquisition annotation may comprise any graphical and/or textual content for describing image content, such as a marker for indicating orientation and/or laterality of anatomy.

The methods and systems described herein may improve the workflow by automating marker placement and/or size adjustment. Automated marker placement as described may ensure that the marker is located at a configurable, view- and anatomy-dependent location, avoiding overlap with relevant anatomy and/or foreign objects, while ensuring readability. Automated marker sizing as described herein may ensure that the marker when displayed is not too large (which can be distracting, particularly in the case of paediatric examinations or examinations of small body parts like fingers or toes) or too small (particularly in the case of stitched images).

According to a second aspect, there is provided a computing system configured to perform the method of the first aspect.

According to a third aspect, there is provided a computer program (product) comprising instructions which, when executed by a computing system, enable or cause the computing system to perform the method of the first aspect.

According to a fourth aspect, there is provided a computer-readable (storage) medium comprising instructions which, when executed by a computing system, enable or cause the computing system to perform the method of the first aspect. The computer-readable medium may be transitory or non-transitory, volatile or non-volatile.

The term "obtaining", as used herein, may comprise, for example, receiving from another system, device, or process; receiving via an interaction with a user; loading or retrieving from storage or memory; measuring or capturing using sensors or other data acquisition devices.

The term "determining", as used herein, encompasses a wide variety of actions, and may comprise, for example, calculating, computing, processing, deriving, investigating, looking up (e.g., looking up in a table, a database or another data structure), ascertaining, and the like. Also, "determining" may comprise receiving (e.g., receiving information), accessing (e.g., accessing data in a memory), and the like. Also, "determining" may comprise resolving, selecting, choosing, establishing and the like.

The indefinite article "a" or "an" does not exclude a plurality. In addition, the articles "a" and "an" as used herein should generally be construed to mean "one or more" unless specified otherwise or clear from the context to be directed to a singular form.

Unless specified otherwise, or clear from the context, the phrases "one or more of A, B and C", "at least one of A, B, and C", and "A, B and/or C" as used herein are intended to mean all possible permutations of one or more of the listed items. That is, the phrase "A and/or B" means (A), (B), or (A and B), while the phrase "A, B, and/or C" means (A), (B), (C), (A and B), (A and C), (B and C), or (A, B, and C).

The term "comprising" does not exclude other elements or steps. Furthermore, the terms "comprising", "including", "having" and the like may be used interchangeably herein.

The invention may include one or more aspects, examples or features in isolation or combination whether specifically disclosed in that combination or in isolation. Any optional feature or sub-aspect of one of the above aspects applies as appropriate to any of the other aspects.

The above-described aspects will become apparent from, and elucidated with, reference to the detailed description provided hereinafter.

### BRIEF DESCRIPTION OF THE DRAWINGS

A detailed description will now be given, by way of example only, with reference to the accompanying drawings, in which:
Fig. 1 illustrates manually-placed markers in medical images;
Fig. 2 shows two medical images viewed at different zoom factors;
Fig. 3 illustrates steps of an algorithm for automatic marker placement and scaling in medical images; and
Fig. 4 illustrates a computing system that can be used in accordance with the systems and methods disclosed herein.

### DETAILED DESCRIPTION OF EMBODIMENTS

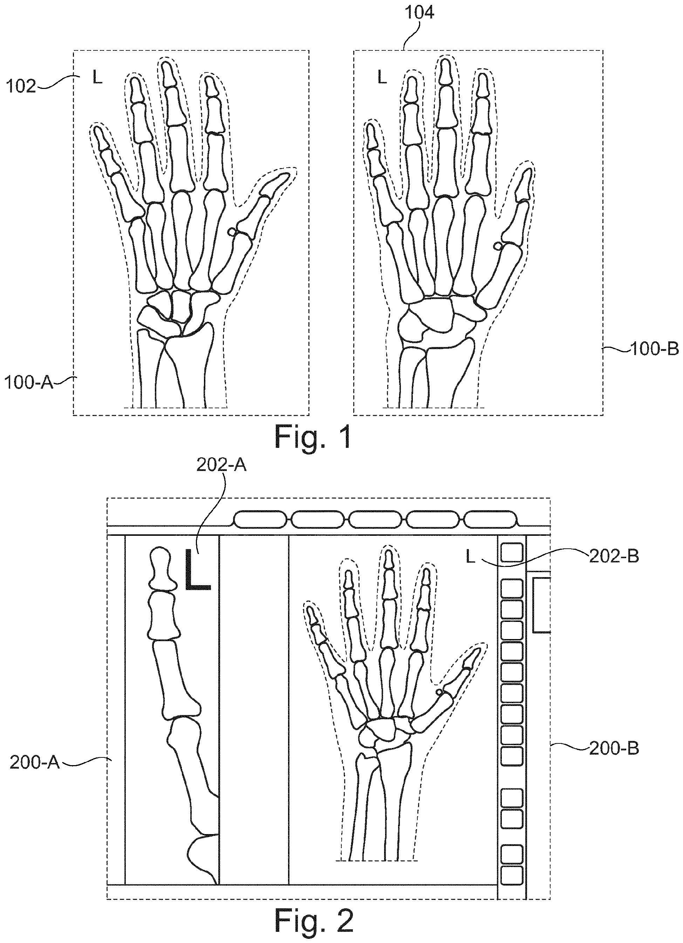

Fig. 1 illustrates two medical images 100 to which manually-placed markers have been applied. In the left-hand image 100-A, the radiographer has manually placed a post-acquisition marker 102 using a software package to indicate left laterality of the patient's hand. In the right-hand image 100-B, the radiographer has placed a pre-acquisition marker (a lead marker) 104 on the detector alongside the patient's hand to indicate left laterality. Since lead is a radiopaque material, the marker 104 appears in the acquired image.

Fig. 2 shows two medical images 200 viewed at different zoom factors. The left-hand image 200-A shows a finger examination and the right-hand image 200-B shows a hand examination. In each image 200, a marker 202 is placed to indicate left laterality. Medical images are typically viewed using fit-to-screen scaling. Therefore, the marker 202-A in the finger image 200-A is larger than the marker 202-B in the hand image 202-B, although the same marker size (with respect to the detector pixel matrix) was selected. This effect becomes more pronounced when the same marker configuration is used for a paediatric finger examination and for large stitching examinations spanning several detector heights.

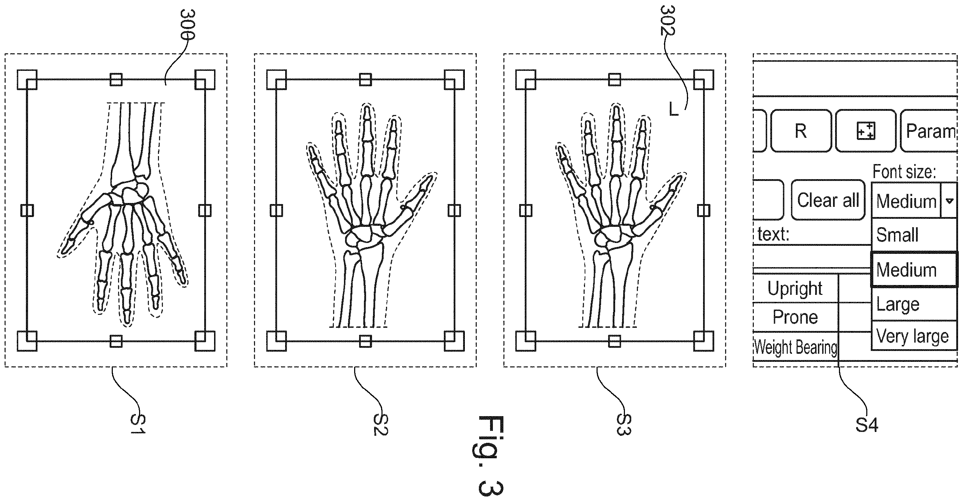

Fig. 3 illustrates steps of an algorithm for automatic marker placement and scaling in medical images according to the present disclosure, which serves to improve the radiology workflow and reduce the overall time for patient examination.

In step S 1, a medical image 300 to be annotated is obtained. In the non-limiting example shown, the image 300 is an X-ray image of a patient's (left) hand.

In step S2, the medical image 300 is optionally pre-processed. In the non-limiting example illustrated in Fig. 3, the pre-processing comprises a rotation of the image 300. It will be appreciated that other pre-processing techniques may be applied to the image 300, such as other geometric transformations; noise reduction, contrast enhancement, image resizing, segmentation, or feature extraction, for example.

In subsequent steps, an annotation algorithm is applied to the medical image 300 to determine values of one or more parameters for a post-acquisition annotation to be applied to the image.

In step S3, the annotation algorithm determines image coordinates for a position of the post-acquisition annotation in the medical image 300. Fig. 3 shows the medical image 300 annotated by application of a left laterality marker 302 to the image using image coordinates which place the marker 302 in the upper-right hand corner of the image 300. Step S3 may comprise manual or automatic selection of the correct marker. For example, in case that the laterality is not known, automatic detection of the laterality, and thereby of the correct marker, may be performed using one or more techniques as known in the art.

In step S4, the annotation algorithm optionally resizes the marker 302 using a determined scaling factor to achieve a desired appearance. For example, returning to Fig. 2, the scaling factor may be determined such that the marker 302 appears with an appropriate size (that is, within predefined constraints) at multiple different zoom factors. In a variant, in which the algorithm is executed at a viewing station at which the image is viewed, the zoom factor selected for display at the viewing station may be used by the annotation algorithm as an additional input for the marker size determination, for example such that the marker 302 appears at a consistent size relative to screen size at multiple different zoom factors. In the non-limiting example shown in Fig. 3, the marker size is automatically selected from four different available marker sizes. The marker size may be selected based at least partially on a type of the marker, e.g., laterality, orientation, and so on. Furthermore, the annotation algorithm optionally modifies the brightness of the marker 302 in dependence on the background brightness of the medical image 300.

The algorithm concludes with the annotated medical image 300 being output for display or storage. The determined values can be stored together with the marker 302 (e.g., "L" or "R") in the X-ray image metadata, e.g., DICOM (Digital Imaging and COmmunication in Medicine) presentation state, for later display. Alternatively, the marker 302 can be rendered directly into the X-ray image 300 (i.e., "burned in") using the determined values.

The annotation algorithm may be implemented in different ways. The present disclosure proposes to use an AI-based approach and/or a conventional image processing approach.

In one example of an end-to-end approach, end-to-end training is applied by using a training dataset comprising X-ray images and known (e.g., from image metadata) or annotated marker positions to train a machine learning model to output the image coordinates. The DICOM presentation state may include information about marker position and/or size. Any appropriate model training technique can be used to exploit this information. A model may be trained for each hospital, so that the model learns hospital-specific annotation conventions.

In one example of a conventional image processing approach, the annotation algorithm is configured to select a default initial position for the annotation following manual or automatic anatomy rotation. Depending on the anatomy, examination type and collimation size, the annotation algorithm selects a default marker size, e.g., a font size, and adjusts the position and/or font size to avoid overlap with diagnostically-relevant information and/or foreign objects appearing in the medical image 300. The annotation algorithm may further utilize additional image content information like areas of direct radiation, soft tissue, and bone contours to further adjust the marker position and/or size.

In one example of a model-based approach, model-based quality assurance software, which uses a 2D atlas or a 3D articulated model for determining pose, collimation, and/or posture issues, is extended to perform marker placement with reference to landmarks appearing in the atlas or the model. Using a forward projection of the adapted model, the annotation algorithm determines a suitable marker position within the X-ray image. Prior knowledge about the body part, the orientation and laterality may be considered.

In any of the examples described herein, the annotation algorithm may check entropy within the X-ray image prior to placing the annotation to prevent the annotation being placed on top of diagnostically relevant structures and/or facilitate placement of the annotation in a low entropy areas indicative of a flat region, e.g., a region within the direct radiation area of the image.

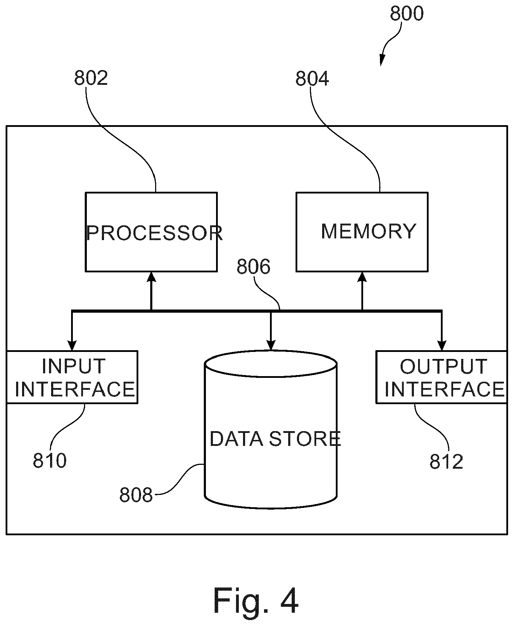

Fig. 4 illustrates an exemplary computing system 800 that can be used in accordance with the systems and methods disclosed herein. The computing system 800 may form part of or comprise any desktop, laptop, server, or cloud-based computing system. The computing system 800 includes at least one processor 802 that executes instructions that are stored in a memory 804. The instructions may be, for instance, instructions for implementing functionality described as being carried out by one or more components described herein or instructions for implementing one or more of the methods described herein. The processor 802 may access the memory 804 by way of a system bus 806. In addition to storing executable instructions, the memory 804 may also store conversational inputs, scores assigned to the conversational inputs, etc.

The computing system 800 additionally includes a data store 808 that is accessible by the processor 802 by way of the system bus 806. The data store 808 may include executable instructions, log data, etc. The computing system 800 also includes an input interface 810 that allows external devices to communicate with the computing system 800. For instance, the input interface 810 may be used to receive instructions from an external computer device, from a user, etc. The computing system 800 also includes an output interface 812 that interfaces the computing system 800 with one or more external devices. For example, the computing system 800 may display text, images, etc. by way of the output interface 812.

It is contemplated that the external devices that communicate with the computing system 800 via the input interface 810 and the output interface 812 can be included in an environment that provides substantially any type of user interface with which a user can interact. Examples of user interface types include graphical user interfaces, natural user interfaces, and so forth. For instance, a graphical user interface may accept input from a user employing input device(s) such as a keyboard, mouse, remote control, or the like and provide output on an output device such as a display. Further, a natural user interface may enable a user to interact with the computing system 800 in a manner free from constraints imposed by input device such as keyboards, mice, remote controls, and the like. Rather, a natural user interface can rely on speech recognition, touch and stylus recognition, gesture recognition both on screen and adjacent to the screen, air gestures, head and eye tracking, voice and speech, vision, touch, gestures, machine intelligence, and so forth.

Additionally, while illustrated as a single system, it is to be understood that the computing system 800 may be a distributed system. Thus, for instance, several devices may be in communication by way of a network connection and may collectively perform tasks described as being performed by the computing system 800.

Various functions described herein can be implemented in hardware, software, or any combination thereof. If implemented in software, the functions can be stored on or transmitted over as one or more instructions or code on a computer-readable medium. Computer-readable media include computer-readable storage media. Computer-readable storage media can be any available storage media that can be accessed by a computer. By way of example, and not limitation, such computer-readable storage media can comprise FLASH storage media, RAM, ROM, EEPROM, CD-ROM or other optical disc storage, magnetic disk storage or other magnetic storage devices, or any other medium that can be used to carry or store desired program code in the form of instructions or data structures and that can be accessed by a computer. Disk and disc, as used herein, include compact disc (CD), laser disc, optical disc, digital versatile disc (DVD), floppy disk, and Blu-ray disc (BD), where disks usually reproduce data magnetically and discs usually reproduce data optically with lasers. Further, a propagated signal may be included within the scope of computer-readable storage media. Computer-readable media also includes communication media including any medium that facilitates transfer of a computer program from one place to another. A connection, for instance, can be a communication medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio and microwave are included in the definition of communication medium. Combinations of the above should also be included within the scope of computer-readable media.

Alternatively, or in addition, the functionally described herein can be performed, at least in part, by one or more hardware logic components. For example, and without limitation, illustrative types of hardware logic components that can be used include Field-programmable Gate Arrays (FPGAs), Application-specific Integrated Circuits (ASICs), Application-specific Standard Products (ASSPs), System-on-a-chip systems (SOCs), Complex Programmable Logic Devices (CPLDs), etc.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features.

It has to be noted that embodiments of the invention are described with reference to different categories. In particular, some examples are described with reference to methods whereas others are described with reference to apparatus. However, a person skilled in the art will gather from the description that, unless otherwise notified, in addition to any combination of features belonging to one category, also any combination between features relating to different category is considered to be disclosed by this application. However, all features can be combined to provide synergetic effects that are more than the simple summation of the features.

While the invention has been illustrated and described in detail in the drawings and foregoing description, such illustration and description are to be considered exemplary and not restrictive. The invention is not limited to the disclosed embodiments. Other variations to the disclosed embodiments can be understood and effected by those skilled in the art, from a study of the drawings, the disclosure, and the appended claims.

The mere fact that certain measures are recited in mutually different dependent claims does not indicate that a combination of these measures cannot be used advantageously.
Any reference signs in the claims should not be construed as limiting the scope.

## Claims

1. A computer-implemented method for annotating medical images, the method comprising:
obtaining a medical image (300) to be annotated;
applying an annotation algorithm to the medical image, wherein the annotation algorithm determines values of one or more parameters for a post-acquisition annotation (302) to be applied to the medical image and annotates the medical image by applying the post-acquisition annotation to the medical image using the determined values, wherein the one or more parameters comprise image coordinates for a position of the post-acquisition annotation in the medical image; and
outputting the annotated medical image.

2. The method according to claim 1, wherein the one or more parameters further comprise a scaling factor for the post-acquisition annotation (302).

3. The method according to claim 1 or 2, wherein the annotation algorithm determines the values of the one or more parameters based on a type of the post-acquisition annotation (302).

4. The method according to claim 3, wherein the type of the post-acquisition annotation (302) corresponds to one or more of the following: a laterality marker; a view position marker; an operator ID marker.

5. The method according to any preceding claim, wherein the one or more parameters further comprise a brightness of the post-acquisition annotation (302), and wherein the annotation algorithm determines the brightness based at least partially on a background brightness of the medical image (300).

6. The method according to any preceding claim, wherein the values determined by the annotation algorithm are stored together with the post-acquisition annotation (302) in metadata for the medical image (300).

7. The method according to any of claims 1-5, wherein the annotation algorithm renders the post-acquisition annotation (302) directly into the medical image (300) using the determined values of the one or more parameters.

8. The method according to any preceding claim, wherein the annotation algorithm utilizes a trained machine learning model to determine the values of the one or more parameters.

9. The method according to any preceding claim, wherein the annotation algorithm utilizes a model of anatomy depicted in the medical image (300) to determine the image coordinates for the position of the post-acquisition annotation based at least partially on one or more landmarks comprised in the model.

10. The method according to claim 9, wherein the annotation algorithm further utilizes prior knowledge about an orientation and/or laterality of the anatomy to determine the image coordinates.

11. The method according to any preceding claim, wherein the annotation algorithm utilizes an image processing algorithm to select the values of the one or more parameters which avoid overlap of the post-acquisition annotation (302) with at least one pre-identified region of the medical image (300) which depicts diagnostically relevant structures and/or foreign objects.

12. The method according to any preceding claim, wherein the annotation algorithm identifies at least one region of the medical image (300) which depicts diagnostically relevant structures based on entropy within the medical image.

13. The method according to any preceding claim, wherein the annotation algorithm identifies at least one direct radiation area of the medical image (300) based on entropy within the medical image, wherein the at least one direct radiation area comprises suitable image coordinates for the position of the post-acquisition annotation (302).

14. A computing system (800) configured to perform the method of any preceding claim.

15. A computer-readable medium (804, 808) comprising instructions which, when executed by a computing system (800), cause the computing system to perform the method of any of claims 1-13.
